# EUROPÄISCHE PATENTSCHRIFT

(11) **EP 0 331 071 B1**
(45) Veröffentlichungstag und Bekanntmachung des Hinweises auf die Patenterteilung: **22.12.1993**
(21) Anmeldenummer: 89103394.6
(22) Anmeldetag: 27.02.1989
(51) Int. Cl.: C08K 3/00, C08L 33/02, C08L 35/00, A61L 25/00, C08J 9/08

(54) **Formkörper aus verformbarem Knochenersatzmaterial**
Mouldings obtainable from mouldable artificial bone material
Masse à mouler à partir d'un materiau modelable pour os

(30) Priorität: 29.02.1988 DE 3806448
(43) Veröffentlichungstag der Anmeldung: 06.09.1989
(73) Patentinhaber: THERA Patent GmbH & Co. KG Gesellschaft für industrielle Schutzrechte, D-82229 Seefeld (DE)
(72) Erfinder: Purrmann, Robert, Dr., D-8130 Starnberg (DE); Guggenberger, Rainer, Dr., D-8031 Hechendorf (DE); Pieper, Günter, D-8031 Seefeld (DE)
(74) Vertreter: Abitz, Walter, Dr.-Ing.

(56) Entgegenhaltungen:
- EP-A- 0 023 013
- Encyclop. of polymer science and engineering; vol. 2, 1985, S. 438-439;
- Encyclop. of chem. technology; vol. 11, 1981, S. 93

## Beschreibung

Die Erfindung betrifft Formkörper aus verformbare Knochenersatzmaterial.

Bei chirurgischen Eingriffen sieht sich der behandelnde Arzt oft vor das Problem gestellt, natürliche Knochendefekte oder bei der Operation entstandene Knochendefekte ausgleichen zu müssen. Zum Einsatz kommen als Knochenersatzmaterial vor allem bioinerte oder bioaktive Materialien, die unter den Begriffen "Biokeramiken, Biogläser und Bioglaskeramiken" bekannt sind. Unter "bioinert" versteht man hierbei bisher Materialien, die keine Gewebereaktion auslösen und keine Fremdstoffe abgeben. Hierzu zählen kann man auch Implantationskörper aus Titan, welche an der Oberfläche eine Schicht von Titanoxid haben, die in diesem Fall die Aufgabe der bioinerten Keramikschicht erfüllt. Als "bioaktiv" bezeichnet man nach heutigem Sprachgebrauch Materialien, die die Eigenschaft besitzen, direkt mit Knochengewebe zu verwachsen. Hierzu zählen Biogläser, Bioglaskeramiken, die an der Oberfläche deutliche Anteile von Calciumphosphatkeramiken haben, wie Hydroxylapatit- und Tricalciumphosphatkeramiken. Als Knochenersatzmaterial finden somit Anwendung verschiedene Hydroxylapatit- und Tricalciumphosphatkeramiken, die in granulierter Form sowie in vorgefertigten Formkörpern angeboten werden. Die Herstellung der Materalien erfordert recht aufwendige Sinterprozesse, bei denen über Temperatur und bestimmte Zuschlagstoffe eine Mikro- und Makroporosität erreicht werden kann.

Weiterhin ist bekannt, zur Bekämpfung von Knochendefekten Implantationsmaterialien auf Basis von Polyacrylaten (z.B. PMMA) einzusetzen, die mit Füllstoffpartikeln auf Basis Tricalciumphosphat gefüllt sind (Deutsche Offenlegungsschrift 33 25 111). In der Offenlegungsschrift wird auch beschrieben, dass es vorteilhaft sein kann, neben dem Tricalciumphosphat weitere körperverträgliche resorbierbare Stoffe zuzusetzen. Eine Porosität an der Oberfläche des Ersatzmaterials wird hierbei durch die Resorption erzeugt und soll das Einwachsen von Knochenmaterial erleichtern.

In ähnlicher Weise beschreibt die Deutsche Offenlegungsschrift 27 52 297 gefüllte Polymethylmethacrylat-Materialien, die neben einem resorbierbaren Füllstoff (z.B. Na₂HPO₄) Carbonate und Phosphorsäure enthalten. Durch das Zusammenwirken von Phosphorsäure und Carbonat entsteht beim Anmischen über einen Schäumungsprozess eine poröse Materialstruktur. Da die Materialien aber flüssige Monomere und Phosphorsäure enthalten, sind sie toxikologisch nicht unbedenklich. Zudem ist bei der Abbindereaktion mit hohen Temperaturen zu rechnen, wobei angrenzendes Gewebe zerstört werden kann. Eine "bioaktive" Wirkung ist angesichts dieser Tatsache wohl kaum möglich.

Im Bereich der Zahnmedizin werden seit längerer Zeit sogenannte "Glasionomerzemente" eingesetzt. Hier handelt es sich um Reaktionsprodukte aus einem Aluminiumfluorosilikatglaspulver mit einer wasserlöslichen Polycarbonsäure und Wasser. Diese Materialien werden vor allem als Zahnfüllmaterial, Zahnzementmaterial angewendet, auch der Einsatz als Knochenzement, also zum Befestigen von Prothesen im Knochen wurde beschrieben (siehe z.B. Deutsche Offenlegungsschrift 29 29 121).

Aufgabe der Erfindung ist die Bereitstellung von leicht herzustellenden, Formkörpern, welche keine toxikologisch bedenklichen, niedermolekularen Monomeren enthalten

Gegenstand der Erfindung ist ein Formkörper mit durchgehend miteinander verbundenen Poren, erhältlich durch Vermischen der Bestandteile (a), (b), (c) und gegebenenfalls (d) und/oder (e) eines Verformbaren Materials und Härten, wobei das verformbare Material
(a) ein Aluminiumfluorosilikatglas,
(b) mindestens eine Polycarbonsäure mit einem mittleren Molekulargewicht > 500,
(c) ein Carbonat und/oder Hydrogencarbonat in einer Menge von mindestens 0,1 Gew.-%, bezogen auf (a),
(d) gegebenenfalls ein chelatbildendes Mittel und
(e) gegebenenfalls Wasser,
enthält

Das verformbare Material eignet sich insbesondere als Knochenersatzmaterial.

Durch Zusatz von Wasser erhärtet das nicht-wasserhaltige verformbare Material unter Verschäumung, wobei gleichzeitig eine Formgebung vorgenommen werden kann.

Das wasserhaltige verformbare Material kann in mindestens zwei räumlich voneinander getrennten Teilmassen vorliegen, von denen vorzugsweise mindestens eine Teilmasse fest, vorzugsweise pulverförmig, und mindestens eine weitere Teilmasse flüssig oder pastös ist.

Als feste, vorzugsweise pulverförmige, und als entsprechende flüssige Teilmassen werden vorzugsweise folgende Mischungen eingesetzt:

| feste Teilmasse (Bestandteile) | flüssige Teilmasse (Bestandteile) |
|---|---|
| (a) + (c) | H₂O + (b), ggf. (d) |
| (a) + (c) + (b) | H₂O, ggf. (d) |
| (a) + (c) + (d) | H₂O + (b) |

Gibt man zur Verarbeitung des verformbaren Materials feste und flüssige Teilmassen zusammen, so entsteht durch die Reaktion der Polycarbonsäure mit dem Aluminiumfluorosilikatglas ein Formkörper, welcher durch die gleichzeitige Reaktion der Polycarbonsäure mit dem Carbonat und/oder Hydrogencarbonat mit Makroporen versehen wird.

In einer bevorzugten Ausführungsform besteht das verformbare Material aus einer flüssigen Teilmasse, die 40 bis 90 Gew.-% Wasser, 10 bis 60 Gew.-% Polycarbonsäure sowie 0 bis 20 Gew.-% Chelatbildner enthält, und aus einer festen Teilmasse, die 80 bis 99,9 Gew.-% Aluminiumfluorosilikatglas und 0,1 bis 20 Gew.-% eines Carbonats und/oder Hydrogencarbonats enthält.

In einer weiteren bevorzugten Ausführungsform besteht das verformbare Material aus einer flüssigen Teilmasse, die 80 bis 100 Gew.-% Wasser sowie 0 bis 20 Gew.-% Chelatbildner enthält, und aus einer festen Teilmasse, die 50 bis 95 Gew.-% Aluminiumfluorosilikatglas, 4,9 bis 50 Gew.-% trockene Polycarbonsäure sowie 0,1 bis 20 Gew.-% Carbonat und/oder Hydrogencarbonat enthält.

Zusätzlich können die verformbaren Materialien z.B. Konservierungsmittel, wie Benzoesäure, sowie Thixotropiehilfsmittel, Füllstoffe, Pigmente u.ä. enthalten.

Die erfindungsgemässen Formkörper können vor ihrer Verwendung an der Oberfläche leicht angeschliffen werden, so dass die erzielten Poren frei zugänglich sind.

Mit den verformbaren Materialien lassen sich die Vorteile gut biokompatibler Materialien, wie Hydroxylapatitkeramiken, mit der guten Applizierbarkeit von gefüllten Polyacrylaten verbinden, ohne hierbei die Nachteile, wie Zusatz von resorbierbaren Substanzen, Einsatz von starken Säuren, Einsatz von toxikologisch bedenklichen Monomeren und schwierige Formgebung harter Keramiken und Gläser, in Kauf nehmen zu müssen.

Mit den Materialien gemäss der Erfindung lassen sich für den Einsatz als Knochenersatz pastöse Massen anmischen, die vom Chirurgen während der Operation einfach und ohne Bearbeitung mit spanabhebenden Werkzeugen in die gewünschte Form gebracht werden können oder direkt in die Knochendefekte eingefüllt werden können, wobei dann die Erhärtung im Knochen erfolgt. Überraschend werden so gut verträgliche Knochenersatzteile erhalten, die aufgrund ihrer Porosität vom Knochenmaterial gut durchwachsen werden können und bei der Abbindereaktion weder durch Säureeinwirkung noch durch Temperaturspitzen das umgebende Knochenmaterial schädigen.

Ein weiterer Vorteil gemäss der vorliegenden Erfindung ist es, dass sich durch die Korngrösse und Menge der eingesetzten Carbonate und/oder Hydrogencarbonate und durch die Wahl der Löslichkeit der Carbonate und der eingesetzten Polycarbonsäure die Makroporosität beeinflussen lässt. Es lassen sich so in idealer Weise Knochenersatzteile herstellen, die in ihrer Porosität dem umgebenden Knochenmaterial, insbesondere der Spongiosa, gleichen.

Ein weiterer Vorteil der verformbaren Materialien ist es, dass die Einzelkomponenten vordosiert in Ausbringvorrichtungen (z.B. Kapseln), wie sie beispielsweise in der Deutschen Offenlegungsschrift 34 07 648 beschrieben sind, angewendet werden können. In diesen Ausbringvorrichtungen befindet sich die pulverförmige Komponente in der Regel im Kapselinnenraum, während sich die flüssige Komponente in einem Kissen an der Kapselwand befindet. Vor der Anwendung wird - mit einem speziellen Aktivator - der Inhalt der Kissenflüssigkeit durch ein Loch in der Kapselwand ins Kapselinnere gepresst. Durch Schütteln wird der Inhalt homogen vermischt. Das Material kann aus der Kapsel direkt in die Knochenöffnung eingebracht werden. Bei dieser Anwendungsweise ist es vorteilhaft, wenn Glas und Carbonat und/oder Hydrogencarbonat als Pulver im Kapselinnenraum aufbewahrt werden und die wässrige Polycarbonsäurelösung und ggf. der Chelatbildner als Flüssigkeitskomponente im Kissen enthalten sind.

Als Bestandteil (a) können die in der Deutschen Offenlegungsschrift 20 61 513 und der Europäischen Patentveröffentlichung 0 023 013 beschriebenen Calciumaluminiumfluorosilikatgläser und die in der Europäischen Patentveröffentlichung 0 241 277 beschriebenen Strontiumaluminiumfluorosilikatgläser eingesetzt werden. Die erfindungsgemäss verwendeten Aluminiumfluorosilikatglas-Pulver bestehen neben Sauerstoff vorzugsweise aus:

| Bestandteil | Berechnet als | Gew.-% |
|---|---|---|
| Si | SiO₂ | 20 - 60 |
| Al | Al₂O₃ | 10 - 50 |
| Ca | CaO | 0 - 40 |
| Sr | SrO | 0 - 40 |
| F | F | 1 - 40 |
| Na | Na₂O | 0 - 10 |
| P | P₂O₅ | 0 - 10 |

wobei mindestens 1 Gew.-% CaO und/oder SrO enthalten sein müssen und insgesamt 0 bis 20 Gew.-%, berechnet als Oxide, an B, Bi, Zn, Mg, Sn, Ti, Zr, La oder anderen 3-wertigen Lanthanoiden, K, W, Ge sowie weiteren Zusätzen, welche die Eigenschaften nicht beeinträchtigen und physiologisch unbedenklich sind. Durch Zusatz von 10 bis 20 Gew.-% La₂O₃ können die Gläser röntgensichtbar gemacht werden.

Vorteilhaft bestehen die Pulverteilchen aus:

| | |
|---|---|
| Si als SiO₂ | 25 - 50 Gew.-% |
| Al als Al₂O₃ | 10- 40 Gew.-% |
| Ca als CaO | 0 - 35 Gew.-% |
| Sr als SrO | 0 - 35 Gew.-% |
| F | 5 - 30 Gew.-% |
| Na als Na₂O | 0 - 8 Gew.-% |
| P als P₂O₅ | 1 - 10 Gew.-% |

wobei mindestens 10 Gew.-% Ca (berechnet als CaO) und/oder Sr (berechnet als SrO) enthalten sein müssen und 0 - 10 Gew.-% an B₂O₃, Bi₂O₃, ZnO, MgO, SnO₂, TiO₂, ZrO₂, La₂O₃ oder anderen Oxiden 3-wertiger Lanthanoide, K₂O, WO₃, GeO₂ sowie weiteren Zusätzen, welche die Eigenschaften nicht beeinträchtigen und physiologisch unbedenklich sind.

Besonders bevorzugt verwendete Pulver enthalten

| | |
|---|---|
| Si als SiO₂ | 25 - 45 Gew.-% |
| Al als Al₂O₃ | 20 - 40 Gew.-% |
| Ca als CaO | 10 - 30 Gew.-% |
| F | 10 - 30 Gew.-% |
| Na als Na₂O | 1 - 8 Gew.-% |
| P als P₂O₅ | 1 - 10 Gew.-%. |

Die erfindungsgemäss verwendeten Glaspulver weisen eine durchschnittliche Korngrösse (Gewichtsmittel) von wenigstens 1 µm und bevorzugt mindestens 3 µm auf. Die durchschnittliche Korngrösse (Gewichtsmittel) beträgt 1 - 20 µm, bevorzugt 3 - 15 µm und besonders bevorzugt 3 - 10 µm. Die Teilchen haben eine maximale Korngrösse von 150 µm, vorzugsweise 100 µm, besonders 60 µm.

Die so erhaltenen Pulver werden dann gegebenenfalls einer Oberflächenbehandlung gemäss der europäischen Patentschrift 0 023 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bzw. Strontiumsalze bilden. Die Säuren werden in einer Konzentration von 0,01 bis 10 Gew.-%, vorzugsweise von 0,05 bis 3 Gew.-% eingesetzt. Nach der entsprechenden Reaktionszeit werden die Pulver von der Lösung separiert und gründlich ausgewaschen, so dass sich praktisch keine löslichen Calcium- oder Strontiumsalze mehr auf der Oberfläche der Pulverteilchen befinden.

Die als Bestandteil (b) einzusetzenden Polycarbonsäuren können auch die bei der Herstellung von Glasionomerzementpulvern bekannten Polycarbonsäuren, z.B. Polymaleinsäure, Polyacrylsäure, Polyitaconsäure sowie Gemische davon oder Copolymere, insbesondere die aus der Europäischen Patentschrift 0 024 056 bekannten Maleinsäure-Acrylsäure-Copolymeren und/oder Acrylsäure-Itakonsäure-Copolymerensein. Das mittlere Molekulargewicht der erfindungsgemäss einzusetzenden Polycarbonsäuren beträgt mehr als 500. Vorteilhaft ist ein mittleres Molekulargewicht von 1.000 bis 20.000, besonders bevorzugt sind 3.000 - 10.000. Die Polycarbonsäure wird vorzugsweise in Konzentrationen von 5 bis 50 Gew.-%, bezogen auf Bestandteil (a), eingesetzt.

Um hohe Lagerstabilitäten der Knochenersatzmaterialien vor der Anwendung zu erhalten, empfiehlt sich ein Zusatz von Konservierungsstoffen, z.B. Benzoesäure, insbesondere zur trockenen Polycarbonsäure.

Als Bestandteil (d) kann ein chelatbildendes Mittel, wie es in der Deutschen Offenlegungsschrift 23 19 715 beschrieben ist, enthalten sein. Vorzugsweise wird als Chelatbildner Weinsäure eingesetzt.

Als schaumbildender Bestandteil (c) eignet sich alle Carbonate und/oder Hydrogencarbonate; vorzugsweise sind diese in der wässrigen Polycarbonsäurelösung, welche gegebenenfalls auch den Chelatbildner enthält, zumindest teilweise löslich. Bevorzugt eingesetzt werden physiologisch verträgliche Carbonate, wie die Carbonate und/oder Hydrogencarbonate der Alkali- und/oder Erdalkali-Metalle. Besonders bevorzugt sind die Carbonate und Hydrogencarbonate des Magnesiums, Calciums und Strontiums.

Die als schaumbildender Bestandteil (c) einzusetzenden Carbonate und/oder Hydrogencarbonate werden vorzugsweise in Konzentrationen von 0,1 - 20 Gew.-%, bezogen auf Bestandteil (a), eingesetzt; bevorzugt eingesetzt werden 0,5 - 5 Gew.-%, ganz besonders bevorzugt 1 - 3 Gew.-%.

Das Gewichtsverhältnis von Komponente (b) zu Komponente (c) beträgt vorzugsweise mindestens 3 : 1, besonders bevorzugt ist ein Gewichtsverhältnis von mindestens 10 : 1.

Die Carbonate und/oder Hydrogencarbonate haben vorzugsweise eine mittlere Korngrösse von 0,1 - 200 µm, bevorzugt von 1 - 100 µm, ganz besonders bevorzugt von 5 - 50 µm.

Die Löslichkeit der Carbonate und/oder Hydrogencarbonate kann über die Wahl des (der) Kation(en)s gesteuert werden.

Sie sollte so bemessen werden, dass der Schäumungsprozess bis zur beginnenden Abbindung anhält. Bei einer gewünschten schnellen Abbindung empfiehlt sich somit die Wahl von leicht löslichen Alkalicarbonaten und/oder -hydrogencarbonaten, bei langsamer Abbindung die Wahl von schwerer löslichen Carbonaten und/oder Hydrogencarbonaten.

### BEISPIELE

Die in den Beispielen verwendeten Aluminiumfluorosilikatzusammensetzungen sind in Tabelle 1 wiedergegeben.

**Tabelle 1**

| | Gew.-% | |
|---|---|---|
| | A | B |
| Si als SiO₂ | 25,5 | 27,6 |
| Al als Al₂O₃ | 20,5 | 26,0 |
| Ca als CaO | 14 | 28,8 |
| F | 15 | 17,0 |
| Na als Na₂O | 2 | 2,1 |
| P als P₂O₅ | 4 | 8,3 |
| La als La₂O₃ | 20 | 0 |

### Beispiel 1

### Radiopakes, härtbares, pastöses Knochenersatzmaterial

100 Gew.-teile eines Calciumaluminiumfluorosilikatglaspulvers der Zusammensetzung A der Tabelle 1 (durchschnittliche Teilchengrösse 6 µm), werden mit 1 Gew.-teil Calciumcarbonat (Fa. Merck, durchschnittliche Teilchengrösse 40 µm) sowie 20,3 Gew.-teilen eines Copolymeren (1 : 1) aus Acrylsäure und Maleinsäure (durchschnittliches Molekulargewicht 7000) zu einem homogenen Pulver vermischt. (Das trockene Copolymer wird mit 0,9 Gew.-% Benzoesäure, bezogen auf das Copolymer, stabilisiert).

3,4 Gew.-teile dieser so erhaltenen Pulvermischung A werden mit 1 Gew.-teil H₂O dest. homogen innerhalb von einer halben Minute vermischt. Während der Verarbeitungszeit von 5 Minuten (gerechnet vom Anmischbeginn) schäumt das Material auf und wird nach 15 Minuten zu einem festen, geschäumten Formkörper.

Zur Bestimmung der Druckfestigkeiten werden jeweils 5 Probekörper (Zylinder, Durchmesser 4 mm, Höhe 7 mm) in einer Zwick-Universal-Prüfmaschine untersucht. Die Druckfestigkeit beträgt im Mittel 2,5 MPa.

Die gebildeten Formkörper zeigen Porengrössen von 0,1 - 1,5 mm, die untereinander durchgehend verbunden sind.

Die Löslichkeit wird nach ISO/DIS-Spezifikation 7489 für Glasionomerzement bestimmt und beträgt 0,18 %.

Zur Bestimmung der Bioaktivität wurden Probekörper mit den Massen 15 x 5 x 5 mm (2 Stück) und 15 x 10 x 2 mm (2 Stück) in die rechte Fibula eines Pavians implantiert. Nach 2 Wochen zeigten Röntgenaufnahmen von den Implantaten eine überschiessende Knochenbildungsaktivität und nach weiteren 4 Wochen einen vollkommenen Einbau der Probe in neugebildetes Knochenmaterial. Durch die geringe Löslichkeit wird das Material nicht resorbiert, die interpenetrierenden Poren werden vollständig vom neugebildeten Knochenmaterial durchwachsen.

### Beispiel 2

### Radioluzentes, härtbares, pastöses Knochenersatzmaterial

100 Gew.-teile Calciumaluminiumfluorosilikatglas der Zusammensetzung B der Tabelle 1 werden mit 1 Gew.-teil Calciumcarbonat (Fa. Merck, 40 µm) zu einem homogenen Pulver vermischt. 2 Gew.-teile dieses Pulvers werden mit einer Lösung, bestehend aus 35 % Copolymer des Beispiels 1 und 65 % H₂O dest. zu einer homogenen Paste angemischt. Das Material hat eine Verarbeitungszeit von 4 Minuten, 30 Sekunden (gerechnet vom Anmischbeginn) und wird nach 12 Minuten, 30 Sekunden vollständig hart. Das Material schäumt dabei zu einem porösen Formkörper mit Porengrössen zwischen 0,1 und 1,5 mm, die Druckfestigkeit des Materials beträgt 2,6 MPa und die Löslichkeit nach ISO/DIS-Spezifikation 7489 0,19 %.

Von dem Material wurden Probekörper mit den Ausmassen 15 x 20 x 5 mm (2 Stück) in die linke Tibia eines Pavians implantiert. Auf den Röntgenaufnahmen ist das Implantat nur schlecht sichtbar im Vergleich mit dem Knochenmaterial. Nach 2 Wochen zeigt sich am Rand des Implantats eine überschiessende Knochenbildungsaktivität und nach weiteren 4 Wochen ist die Stelle nicht mehr vom umgebenden Knochenmaterial zu unterscheiden, da das Implantat vollkommen vom neugebildeten Knochenmaterial durchwachsen wird.

### Beispiel 3

### In einer Kapsel anmischbares Knochenersatzmaterial

700 mg Calciumaluminiumfluorosilikatglaspulver der Zusammensetzung B der Tabelle 1 und 7 mg Calciumcarbonat (Fa. Merck, mittlere Korngrösse 2,3 µm) werden in den Kapselinnenraum einer handelsüblichen Glasionomerkapsel aus dem Dentalbereich (KETACFIL, Fa. ESPE) eingewogen. An der Kapselaussenwand wird ein Kissen, enthaltend 350 mg einer 35 Gew.-% enthaltenden Copolymerlösung in H₂O dest. befestigt und anschliessend wie die entsprechenden Glasionomerfüllungsmaterialien aktiviert und geschüttelt (Silamat, Fa. Ivoclar, 4300 Schwingungen/Minute, Schüttelzeit 10 Minuten. Das Material hat eine Verarbeitungszeit von 2 Minuten, 10 Sekunden und erhärtet nach 5 Minuten. Die Druckfestigkeit beträgt 7 MPa und die Löslichkeit nach ISO/DIS-Spezifikation 7489 beträgt 0,18 %. Die Poren zeigen eine Grösse von 0,1 bis 1,2 mm und sind durchgehend miteinander verbunden.

## Patentansprüche

1. Formkörper mit durchgehend miteinander verbundenen Poren, erhältlich durch Vermischen der Bestandteile (a), (b), (c) und gegebenenfalls (d) und/oder (e) eines verformbaren Materials und Härten, wobei das verformbare Material
(a) ein Aluminiumfluorosilikatglas,
(b) mindestens eine Polycarbonsäure mit einem mittleren Molekulargewicht > 500,
(c) ein Carbonat und/oder Hydrogencarbonat mit einer mittleren Korngröße von 0,1 bis 200 µm in einer Menge von mindestens 0,1 Gew.-%, bezogen auf (a),
(d) gegebenenfalls ein chelatbildendes Mittel und
(e) gegebenenfalls Wasser
enthält.

2. Formkörper nach Anspruch 1, dadurch gekennzeichnet, daß das verformbare Material vor dem Vermischen in mindestens zwei räumlich voneinander getrennten Teilmassen vorliegt.

3. Formkörper nach Anspruch 2, dadurch gekennzeichnet, daß mindestens eine Teilmasse in fester, vorzugsweise pulverförmiger Form und mindestens eine andere Teilmasse in flüssiger oder pastöser Form vorliegen.

4. Formkörper nach Anspruch 3, dadurch gekennzeichnet, daß die feste Teilmasse die Bestandteile (a) und (b) und die flüssige Teilmasse die Bestandteile (c) und (e) und gegebenenfalls den Bestandteil (d) enthalten.

5. Formkörper nach Anspruch 3, dadurch gekennzeichnet, daß die feste Teilmasse die Bestandteile (a), (b) und (c) und die flüssige Teilmasse die Bestandteile (e) und gegebenenfalls (d) enthalten.

6. Verfahren zur Herstellung der Formkörper nach mindestens einem der Ansprüche 1 bis 5, dadurch gekennzeichnet, daß man die Bestandteile (a), (b) und (c) und gegebenenfalls (d) und/oder (e) miteinander vermischt und anschließend härtet.

## Claims

1. Shaped article with pores forming a communicating network, obtainable by mixing the constituents (a), (b), (c) and optionally (d) and/or (e) of a shapable material and curing, the shapable material containing
(a) an aluminum fluorosilicate glass,
(b) at least one polycarboxylic acid having an average molecular weight > 500,
(c) a carbonate and/or hydrogen carbonate having an average particle size of 0.1 to 200 µm in an amount of at least 0.1% by weight, based on (a),
(d) optionally a chelating agent and
(e) optionally water.

2. Shaped article according to claim 1, characterized in that the shapable material is present prior to mixing in at least two spatially separate part compositions.

3. Shaped article according to claim 2, characterized in that at least one part composition is present in solid, preferably pulverulent, form and at least one other part composition is present in liquid or paste form.

4. Shaped article according to claim 3, characterized in that the solid part composition contains the constituents (a) and (b) and the liquid part composition contains the constituents (c) and (e) and optionally the constituent (d).

5. Shaped article according to claim 3, characterized in that the solid part composition contains the constituents (a), (b) and (c) and the liquid part composition contains the constituents (e) and optionally (d).

6. Method for preparing the shaped articles according to at least one of claims 1 to 5, characterized in that the constituents (a), (b) and (c) and optionally (d) and/or (e) are mixed with each other and subsequently cured.

## Revendications

1. Articles moulés comportant des pores reliés entre eux de part en part, qu'on obtient en mélangeant les constituants (a), (b), (c) et éventuellement (d) et/ou (e) d'un matériau déformable et en les faisant durcir, le matériau déformable contenant :
(a) un verre de fluorosilicate d'aluminium,
(b) au moins un acide polycarboxylique d'un poids moléculaire moyen > 500,
(c) un carbonate et/ou un hydrogénocarbonate d'une grosseur moyenne de grain de 0,1 à 200 µm, en une quantité d'au moins 0,1 % en poids par rapport à (a),
(d) éventuellement un agent chélateur, et
(e) éventuellement de l'eau.

2. Articles moulés selon la revendication 1, caractérisés en ce que le matériau déformable se présente, avant le mélange, sous la forme d'au moins deux masses partielles séparées dans l'espace.

3. Articles moulés selon la revendication 2, caractérisés en ce qu'au moins une masse partielle est à l'état solide, de préférence à l'état pulvérulent, et au moins une autre masse partielle est à l'état liquide ou pâteux.

4. Articles moulés selon la revendication 3, caractérisés en ce que la masse partielle solide contient les constituants (a) et (b), et la masse partielle liquide contient les constituants (c) et (e) et éventuellement le constituant (d).

5. Articles moulés selon la revendication 3, caractérisés en ce que la masse partielle solide contient les constituants (a), (b) et (c), et la masse partielle liquide contient les constituants (e) et éventuellement (d).

6. Procédé de fabrication des articles moulés selon au moins une des revendications 1 à 5, caractérisé en ce qu'on mélange les constituants (a), (b) et (c) et éventuellement (d) et/ou (e) et ensuite, on fait durcir le mélange.
